# EUROPEAN PATENT APPLICATION

(11) **EP 4 491 610 A1**
(43) Date of publication of application: **15.01.2025**
(21) Application number: 23860784.0
(22) Date of filing: 24.08.2023
(51) Int. Cl.: C07C 51/43, C07C 57/04

(54) **METHOD FOR PREPARATION OF HIGH PURITY (METH)ACRYLIC ACID**

(30) Priority: 30.08.2022 KR 20220109527; 16.08.2023 KR 20230107080
(71) Applicant: LG Chem, Ltd., Yeongdeungpo-gu Seoul 07336 (KR)
(72) Inventor: YOO, Sung Jin, Daejeon 34122 (KR); JANG, Kyung Soo, Daejeon 34122 (KR); LEE, Sung Kyu, Daejeon 34122 (KR)
(74) Representative: Goddar, Heinz J.
(86) International application number: PCT/KR2023/012565
(87) International publication number: WO 2024/049107

(57) **Abstract**

Provided is a method for preparation of (meth)acrylic acid including: bringing a mixed gas including (meth)acrylic acid into contact with water in an absorption column to obtain a (meth)acrylic acid aqueous solution; supplying the (meth)acrylic acid aqueous solution to an extraction column, bringing the (meth)acrylic acid aqueous solution into contact with an extraction solvent to perform extraction, and obtaining an extract including the (meth)acrylic acid and the extraction solvent; supplying the extract to a solvent purification column and separating the extract into a lower discharge stream of the solvent purification column including (meth)acrylic acid and an upper discharge stream of the solvent purification column including the extraction solvent in the solvent purification column; supplying the lower discharge stream of the solvent purification column to a crystallizer and obtaining crystallized (meth)acrylic acid in the crystallizer; and supplying a mother liquor to a high-boiling point by-product separation column after the crystallization and circulating an upper discharge stream of the high-boiling point by-product separation column from which high-boiling point by-products have been removed to the solvent purification column.

## Description

### [Technical Field]

### CROSS-REFERENCE TO RELATED APPLICATIONS

The present application claims the benefit of priorities to Korean Patent Application No. 10-2022-0109527, filed on August 30, 2022, and Korean Patent Application No. 10-2023-0107080, filed on August 16, 2023, the entire contents of which are incorporated herein as a part of the specification.

### Technical Field

The present invention relates to a method for preparation of high-purity (meth)acrylic acid.

### [Background Art]

(Meth)acrylic acid is generally prepared by a method of subjecting a compound such as propane, propylene, and (meth)acrolein to a gas phase oxidation reaction in the presence of a catalyst. For example, propane, propylene, and the like are converted through (meth)acrolein into (meth)acrylic acid by a gas phase oxidation reaction in the presence of an appropriate catalyst in a reactor, and a mixed gas including (meth)acrylic acid, unreacted propane or propylene, (meth)acrolein, inert gas, carbon dioxide, water vapor, and various organic by-product by the reaction (such as acetic acid, low-boiling point by-products, and high-boiling point by-products) is obtained in a latter stage of the reactor.

The (meth)acrylic acid-containing mixed gas is brought into contact with an absorption solvent such as water in an absorption column to be recovered as a (meth)acrylic acid aqueous solution. Further, as a subsequent process for recovering the (meth)acrylic acid included in the (meth)acrylic acid aqueous solution, it is common to involve processes such as extraction, distillation, and purification. In order to improve recovery efficiency of the (meth)acrylic acid, various methods of adjusting process conditions, process order, or the like have been suggested.

However, since the absorption solvent such as water used in the absorption column has a high specific heat, significantly high energy usage is demanded in separating by-products from the (meth)acrylic acid aqueous solution including water through a process such as distillation. Meanwhile, when the subsequent process is simplified and shortened for reducing the energy usage, the energy usage may be decreased, but it is difficult to obtain high-purity (meth)acrylic acid.

Furthermore, acetic acid and high-boiling point by-products which are main by-products of a preparation process of (meth)acrylic acid are separated and removed so that they do not accumulate in the system, and in this process, loss of (meth)acrylic acid, which is the desired product, is incurred.

Therefore, it is urgent to introduce a technology which may reduce the energy usage throughout the entire process, while also minimizing loss of high-purity (meth)acrylic acid from a (meth)acrylic acid aqueous solution.

### [Disclosure]

### [Technical Problem]

In order to solve the problems mentioned in the Background Art, an object of the present invention is to provide a method for preparation of (meth)acrylic acid, which may further reduce energy usage in a purification process, while securing a high (meth)acrylic acid recovery rate.

### [Technical Solution]

In one general aspect, a method for preparation of (meth)acrylic acid includes: bringing a mixed gas including (meth)acrylic acid into contact with water in an absorption column to obtain a (meth)acrylic acid aqueous solution; supplying the (meth) acrylic acid aqueous solution to an extraction column, bringing the (meth)acrylic acid aqueous solution into contact with an extraction solvent to perform extraction, and obtaining an extract including the (meth)acrylic acid and the extraction solvent; supplying the extract to a solvent purification column and separating the extract into a lower discharge stream of the solvent purification column including (meth)acrylic acid and an upper discharge stream of the solvent purification column including the extraction solvent in the solvent purification column; supplying the lower discharge stream of the solvent purification column to a crystallizer and obtaining crystallized (meth)acrylic acid in the crystallizer; and supplying a mother liquor to a high-boiling point by-product separation column after the crystallization and circulating an upper discharge stream of the high-boiling point by-product separation column from which high-boiling point by-products have been removed to the solvent purification column.

### [Advantageous Effects]

According to the method for preparation of (meth)acrylic acid according to the present invention, an extraction process is performed before distillation on the entire (meth)acrylic acid aqueous solution discharged from an absorption column to remove water included in the (meth)acrylic acid aqueous solution, while distillation is performed so that an extraction solvent is included in a lower discharge stream of a solvent purification column including (meth)acrylic acid in a subsequent solvent purification process, thereby reducing energy usage required. In addition, high-purity (meth)acrylic acid may be obtained by a crystallization process for the lower discharge stream of the solvent purification column.

Furthermore, since a mother liquor from which most of (meth)acrylic acid has been removed by the crystallization process is distilled to remove high-boiling point by-products, accumulation of high-boiling point by-products in the system is prevented, and energy usage due to a decrease in an amount to be distilled for removing high-boiling point by-products may be reduced.

### [Description of Drawings]

FIG. 1 is a process flowchart showing a method for preparation of (meth)acrylic acid according to an exemplary embodiment of the present invention.
FIGS. 2 to 4 are process flow charts showing methods for preparation of (meth)acrylic acid according to the comparative examples of the present invention.

### [Best Mode]

The terms and words used in the description and claims of the present invention are not to be construed limitedly as having general or dictionary meanings but are to be construed as having meanings and concepts meeting the technical ideas of the present invention, based on a principle that the inventors are able to appropriately define the concepts of terms in order to describe their own inventions in the best mode.

The term "stream" in the present invention may refer to a fluid flow in a process, or may refer to a fluid itself flowing in a pipe. Specifically, the stream may refer to both a fluid itself flowing in a pipe connecting each device and a fluid flow. In addition, the fluid may refer to a gas or liquid, and a case in which a solid substance is included in the fluid is not excluded.

Meanwhile, in the devices such as an absorption column, a degassing column, a distillation column or a distillation column, and a crystallizer, unless otherwise particularly stated, a "lower portion" of the device refers to a point at a height of 95% to 100% from top to bottom of the device, specifically a lowest part (bottom). Likewise, unless otherwise particularly stated, an "upper portion" of the device refers to a point at a height of 0% to 5% from the top to the bottom of the device, specifically the highest part (top).

Hereinafter, the present invention will be described in more detail for better understanding of the present invention.

The method for preparation of (meth)acrylic acid according to an exemplary embodiment of the present invention may include: bringing a mixed gas including (meth)acrylic acid into contact with water in an absorption column to obtain a (meth)acrylic acid aqueous solution; supplying the (meth)acrylic acid aqueous solution to an extraction column, bringing the (meth)acrylic acid aqueous solution into contact with an extraction solvent to perform extraction, and obtaining an extract including the (meth)acrylic acid and the extraction solvent; supplying the extract to a solvent purification column and separating the extract into a lower discharge stream of the solvent purification column including (meth)acrylic acid and an upper discharge stream of the solvent purification column including the extraction solvent in the solvent purification column; supplying the lower discharge stream of the solvent purification column to a crystallizer and obtaining crystallized (meth)acrylic acid in the crystallizer; and supplying a mother liquor to a high-boiling point by-product separation column after the crystallization and circulating an upper discharge stream of the high-boiling point by-product separation column from which high-boiling point by-products have been removed to the solvent purification column.

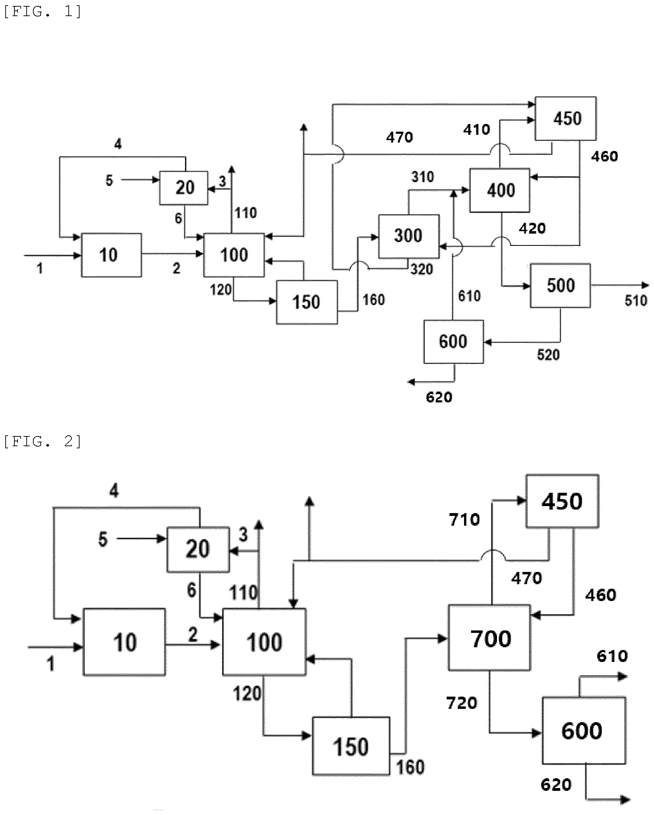

Hereinafter, each process which may be included in the exemplary embodiment of the present invention will be described, with reference to FIG. 1.

First, the method for preparation of (meth)acrylic acid according to an exemplary embodiment of the present invention may include bringing a mixed gas including (meth)acrylic acid into contact with water in an absorption column to obtain a (meth)acrylic acid aqueous solution.

Herein, the mixed gas including (meth)acrylic acid is a concept which collectively refers to gas phase components discharged from a reactor 10 which produces (meth)acrylic acid by a gas phase oxidation reaction. Specifically, the mixed gas may include (meth)acrylic acid, an unreacted raw material compound, (meth)acrolein, inert gas, carbon monoxide, carbon dioxide, water vapor, various organic by-products (acetic acid, low-boiling point by-products, high-boiling point by-product, etc.), and the like. Herein, a "low-boiling point by-product" (light ends) or a "high-boiling point by-product" (heavies) is a kind of by-product which may be produced in preparation and recovery processes of the target (meth)acrylic acid, and may be a compound having a higher or lower molecular weight than the (meth)acrylic acid.

Specifically, the mixed gas including (meth)acrylic acid may be prepared as follows.

First, a reaction gas including gas containing oxygen and a raw material compound is supplied to a reactor 10 provided with a catalyst through a reaction gas supply line 1, and is subjected to a gas phase oxidation reaction in the presence of a catalyst in the reactor 10 to obtain the mixed gas including (meth)acrylic acid.

Herein, the gas containing oxygen may be air. The raw material compound may be one or more compounds selected from the group consisting of propane, propylene, butane, 1-butylene, t-butylene, and (meth)acrolein, and specifically, the raw material compound may include propylene. Meanwhile, the reaction gas supplied to the reactor 10 may further include a recirculation gas which is recovered from an upper portion of the absorption column 100 and recirculated. Therefore, the mixed gas including (meth)acrylic acid may be a reaction product by the gas phase oxidation reaction of a reactant including air, the raw material compound, and the recirculation gas, in the reactor 10.

The recirculation gas may be derived from the upper portion of the absorption column 100 described later. That is, the mixed gas comes into contact with water which is an absorption solvent in the absorption column 100, and a non-condensable gas which is not dissolved in water may be discharged as an upper discharge stream 110 of the absorption column 100. The non-condensable gas may include impurities such as acetic acid, inert gas, an unreacted raw material compound, and a minimum content of (meth)acrylic acid.

That is, when acetic acid is discharged from the upper portion of the absorption column 100, as an amount of discharged acetic acid is increased, a content of (meth)acrylic acid discharged from the upper portion of the absorption column 100 tends to be increased. This means loss of (meth)acrylic acid. As described later, according to the present invention, since acetic acid in the system may be further separated and removed by a solvent purification column and a layer separator after the absorption column, the acetic acid in the system does not need to be discharged forcefully to the upper discharge stream 110 of the absorption column, and specifically, the acetic acid in an amount sufficient to minimize a content of (meth)acrylic acid in the upper discharge stream 110 of the absorption column may be discharged as the upper discharge stream 110. Thus, the content of (meth)acrylic acid which is discharged from the upper portion of the absorption column 100 and lost may be minimized.

Meanwhile, a part 3 of the upper discharge stream 110 of the absorption column may be supplied to a cooling tower 20, and the rest may be supplied to a waste gas incinerator and discarded.

The cooling tower 20 is provided with a water supply line 5 in the upper portion, and water used as an absorption solvent in the absorption column may be supplied from the water supply line 5 into the cooling tower 20. In the cooling tower 20, water may come into contact with the non-condensable gas included in the part 3 of the upper discharge stream 110 of the absorption column. As described above, the non-condensable gas may include acetic acid and a minimal amount of (meth)acrylic acid, and these components are dissolved in the water, which may be discharged in an aqueous solution form as a lower discharge stream of the cooling tower 20. Thereafter, a lower discharge stream 6 of the cooling tower 20 may be supplied to the absorption column 100.

Water needed in the absorption column 100 may be supplied through the water supply line 5 provided in the upper portion of the cooling tower 20. The water may include, specifically, water such as tap water and deionized water, and may include circulation process water introduced from other processes (e.g., water phase recirculated from an extraction process and/or a distillation process). Further, the absorption solvent may include a small amount of organic by-products (e.g. acetic acid) introduced from other processes.

Meanwhile, most of the acetic acid included in the non-condensable gas is removed by a contact with water in the cooling tower 20 by dissolution in water, and gas which is not dissolved in water is discharged through a recirculation gas transfer line 4 provided in the upper portion of the cooling tower 20 as a recirculation gas. The recirculation gas may be supplied to the reactor 10 so that it may be used in the gas phase oxidation reaction for preparation of (meth) acrylic acid which proceeds in the reactor. The recirculation gas may be mixed with the reaction gas and supplied to the reactor, and may be supplied to the reactor through a separate line 4 from the line 1 to which the reaction gas is supplied.

Thereafter, a process for obtaining the (meth)acrylic acid aqueous solution by supplying the mixed gas 2 including (meth)acrylic acid to the absorption column 100 through a reactor discharge line and bringing the gas into contact with water in the absorption column 100 may be performed. Specifically, the mixed gas including (meth)acrylic acid, organic by-products, and water vapor which are produced by a synthesis reaction of (meth)acrylic acid may be brought into contact with water which is the absorption solvent in the absorption column 100 to obtain the (meth)acrylic acid aqueous solution.

Herein, the kind of absorption column 100 may be determined considering contact efficiency of the mixed gas and the absorption solvent and the like, and for example, may be a packed column type absorption column or a multistage tray type absorption column. To the inside of the packed column type absorption column, a filler such as a Rasching ring, a Pall ring, a saddle, a gauze, and a structured packing may be applied.

Further, considering the efficiency of the absorption process, the mixed gas 2 may be supplied to the lower portion of the absorption column 100, and water which is the absorption solvent may be supplied to the upper portion of the absorption column 100.

Meanwhile, the absorption column 100 may be operated at an internal pressure of 1 to 1.5 bar or 1 to 1.3 bar and at an internal temperature of 50 to 100 °C or 50 to 80 °C, considering the condensation conditions of (meth)acrylic acid, a moisture content depending on a saturated water vapor pressure, and the like.

Meanwhile, according to an exemplary embodiment of the present invention, the (meth)acrylic acid aqueous solution is obtained by an absorption process performed in the absorption column 100, and the (meth)acrylic acid aqueous solution may be discharged as the lower discharge stream 120 of the absorption column 100.

Meanwhile, the upper discharge stream of the absorption column 100 may include a non-condensable gas which is not dissolved in water which is the absorption solvent in the absorption column 100, as described above, and the non-condensable gas may include acetic acid, inert gas, an unreacted raw material compound, and a minimal content of (meth)acrylic acid.

Meanwhile, when the amount of acetic acid discharged to the upper portion of the absorption column 100 is increased and exceeds a certain level, the content of the (meth)acrylic acid discharged to the upper portion of the absorption column 100 is increased, and thus, the amount of (meth)acrylic acid lost in the absorption column is also increased. Therefore, the present invention may minimize the content of the (meth)acrylic acid lost in the absorption column, by controlling the amount of acetic acid included in the upper discharge stream of the absorption column 100. That is, the content of acetic acid in the upper discharge stream of the absorption column may be controlled so that the content of the (meth)acrylic acid lost to the upper portion of the absorption column may be minimized. In addition, since acetic acid may be further separated and removed by a solvent purification column and a layer separator after the absorption column described later, a problem that acetic acid accumulates in the system and acts as an impurity may be solved.

From this point of view, a flow rate ratio of acetic acid discharged to the upper portion of the absorption column may be 20 wt% to 80 wt%, specifically 30 wt% to 60 wt%, based on a flow rate of the acetic acid introduced to the absorption column. Meanwhile, the content of the (meth)acrylic acid included in the upper discharge stream of the absorption column may be 0.1 wt% to 0.5 wt%, specifically 0.2 wt% to 0.3 wt%. Meanwhile, the (meth)acrylic acid aqueous solution may be discharged through an absorption column discharge line 120 and supplied to an extraction column 300. The absorption column discharge line 120 may be provided in a lower portion of the absorption column 100.

According to an exemplary embodiment of the present invention, the (meth)acrylic acid aqueous solution may be directly supplied to the extraction column 300, and supplied to the extraction column 300 via a degassing column 150. Specifically, the (meth)acrylic acid aqueous solution is supplied to the degassing column 150 and a discharge stream 160 of the degassing column from which low-boiling point by-products including acrolein have been removed may be supplied to the extraction column. The acrolein may be a raw material for preparing (meth)acrylic acid and may occur as a by-product during a gas phase oxidation reaction for preparing (meth)acrylic acid. In the degassing column 150, a gas phase fraction including the low-boiling point by-products may be circulated to the absorption column 100, and the (meth)acrylic acid aqueous solution in which the low-boiling point by-products have been degassed may be discharged from a lower portion of the degassing column 150 and introduced to a process for obtaining purified (meth)acrylic acid.

The method for preparation of (meth)acrylic acid according to an exemplary embodiment of the present invention may include supplying the (meth)acrylic acid aqueous solution, specifically the (meth)acrylic acid aqueous solution degassed through the degassing column to the extraction column and bringing the (meth)acrylic acid aqueous solution into contact with an extraction solvent to perform extraction to obtain an extract including the (meth)acrylic acid and the extraction solvent.

The extraction process performed in the extraction column 300 of the present invention may remove most of water included in the (meth)acrylic acid aqueous solution without using a large amount of energy, and supply it to a solvent purification column 400. In this respect, it is preferred that extraction in the extraction column 300 uses a liquid-liquid contact method in terms of improving energy efficiency of the entire process. In addition, the present invention may reduce energy usage required in the distillation process by performing distillation so that the extraction solvent is included in the lower discharge stream 420 of the solvent purification column including (meth)acrylic acid in a subsequent solvent purification process after the extraction column.

Herein, the extraction solvent may be a hydrocarbon solvent which forms an azeotrope with water and an organic by-products (acetic acid, etc.) and does not form an azeotrope with (meth)acrylic acid, but may sufficiently extract (meth)acrylic acid, and also, it is advantageous in the extraction process that its boiling point is 10 to 120°C. Specifically, the extraction solvent may be one or more solvents selected from the group consisting of benzene, toluene, xylene, n-heptane, cycloheptane, cycloheptene, 1-heptene, ethyl-benzene, methyl-cyclohexane, n-butyl acetate, isobutyl acetate, isobutyl acrylate, n-propyl acetate, isopropyl acetate, methyl isobutyl ketone, 2-methyl-1-heptene, 6-methyl-1-heptene, 4-methyl-1-heptene, 2-ethyl-1-hexene, ethylcyclopentane, 2-methyl-1-hexene, 2,3-dimethylpentane, 5-methyl-1-hexene, and isopropyl-butyl-ether.

Meanwhile, it is advantageous in the process that a temperature of the (meth)acrylic acid aqueous solution in the extraction process is 10 to 70°C. Further, it is advantageous in the process that a weight ratio of the extraction solvent to the (meth)acrylic acid aqueous solution in the extraction process is 1:1 to 1:5, preferably 1:1.2 to 1:2.5.

In addition, as the extraction column 300, an extraction device according to the liquid-liquid contact method may be used. A non-limiting example of the extraction device may include a Karr reciprocating plate column, a rotary-disk contactor, a Scheibel column, a Kuhni column, a spray extraction column, a packed extraction column, a pulsed packed column, a bank of mixer-settler, mixer and centrifuge (centrifugal counter current extractor), and the like.

In this way, water in the (meth)acrylic acid aqueous solution discharged from the absorption column 100 is removed, an extract from which (meth)acrylic acid is extracted by the extraction solvent is obtained, and the extract may be supplied to the solvent purification column 400 as an extract stream 310. Specifically, the extract may include acetic acid, (meth)acrylic acid, the extract solvent, and high-boiling point by-products.

In addition, water included in the (meth)acrylic acid aqueous solution may be recovered as a raffinate through the extraction process. The recovered raffinate may be discharged as a raffinate stream 320 and introduced to a layer separator 450 described later.

Subsequently, the method for preparation of (meth)acrylic acid of the present invention may include supplying the extract to the solvent purification column and separating the extract into a lower discharge stream of the solvent purification column including (meth)acrylic acid and an upper discharge stream of the solvent purification column including the extraction solvent in the solvent purification column.

Meanwhile, the content of the (meth)acrylic acid included in the lower discharge stream 420 of the solvent purification column may be 85 wt% to 98 wt%, specifically 90 wt% to 95 wt%. Thus, high-purity purified (meth)acrylic acid may be obtained by the crystallization process from the lower discharge stream 420 of the solvent purification column.

In addition, the lower discharge stream 420 of the solvent purification column may include the extraction solvent, and the content of the extraction solvent may be 1 to 10 wt%, specifically 5 to 10 wt%. When the content of the extraction solvent included in the lower discharge stream 420 of the solvent purification column is 1 wt% or more, energy usage consumed in the solvent purification column 400 may be reduced. When the content is 10 wt% or less, high-purity purified (meth)acrylic acid may be obtained by the crystallization process performed in the crystallizer 500, while also energy required to recirculate the extraction solvent and (meth)acrylic acid in the high-boiling point by-product separation column 600 may be reduced.

Meanwhile, the lower discharge stream 420 of the solvent purification column may include the (meth)acrylic acid and the extraction solvent at the above contents, and include other components such as high-boiling point by-products as a remaining balance. The content of the extraction solvent as such is different from the conventional technology of purifying all of the extraction solvent through the solvent purification column, and also is different from the content of the extraction solvent in the case in which all of the extraction solvent is intended to be distilled, but some extraction solvent remains.

That is, the present invention may remove water included in the (meth)acrylic acid aqueous solution by supplying both the (meth)acrylic acid aqueous solution discharged from the absorption column 100 or the (meth)acrylic acid aqueous solution passing through the degassing column 150 to the extraction column 300, and may reduce energy usage required in the distillation process by performing distillation so that the extraction solvent is included in the lower discharge stream of the solvent purification column including (meth)acrylic acid in the subsequent solvent purification process. When the extraction solvent is removed overall or as much as possible to the upper portion of the solvent purification column 400, the amount of (meth)acrylic acid lost with the extraction solvent may be increased, and thus, a reflux flow rate of the upper portion of the solvent purification column 400 should be increased, but when the lower discharge stream 420 of the solvent purification column includes a certain amount of extraction solvent as in the present invention, loss of (meth)acrylic acid may be minimized, while also the reflux flow rate of the upper portion of the solvent purification column 400 may be decreased, and thus, energy usage required in the solvent purification column 400 may be reduced. That is, by not distilling all of the extraction solvent in the solvent purification column 400, energy usage in the solvent purification column may be reduced as compared with the case of distilling all of the extraction solvent. As such, even in the case in which only a part of the extraction solvent is distilled in the solvent purification column, a residual extraction solvent may be separated through a subsequent crystallization process 500.

For this purpose, an operating temperature in the solvent purification column may be 30°C to 120°C, specifically 40°C to 100°C.

Meanwhile, the upper discharge stream 410 of the solvent purification column 400 and the raffinate including water, that is, the lower discharge stream 320 of the extraction column in the extraction process described above may be introduced to a layer separator 450.

Herein, the layer separator 450 is a liquid-liquid layer separator and a device for separating fluids which are not mixed with each other by a density difference using gravity, centrifugal force, or the like, in which a relatively light liquid may be separated to the upper portion of the layer separator 450 and a relatively heavy liquid may be separated to the lower portion of the layer separator 450. Specifically, the streams supplied to the layer separator 450 may be separated into an organic layer 460 including the extraction solvent and a water layer 470 including water and acetic acid.

In addition, at least a part of the organic layer separated in the layer separator 450 may be supplied to the upper portion of the solvent purification column 400 and reused as a reflux liquid, and the rest may be supplied to the extraction column 300 and reused as the extraction solvent.

According to an exemplary embodiment of the present invention, a ratio of a flow rate of the extraction solvent in a part of an organic layer supplied to the solvent purification column to a flow rate of the extraction solvent in the upper discharge stream 410 of the solvent purification column may be 0.3 to 0.6. When the ratio of the flow rate of the extraction solvent in a part of an organic layer supplied to the solvent purification column to the flow rate of the extraction solvent in the upper discharge stream of the solvent purification column is controlled to the above range, a reflux flow rate of the upper portion of the solvent purification column is low, and thus, the amount of energy used in the solvent purification column 400 may be reduced.

Meanwhile, at least a part of the water layer separated in the layer separator 450 may be supplied to the upper portion of the absorption column 100 and used as an absorption solvent, and the rest may be discharged as waste water.

That is, according to an exemplary embodiment of the present invention, acetic acid may be discharged through the upper discharge stream of the absorption column 100, and also discharged through the solvent purification column 400 and the layer separator 450 simultaneously. Therefore, acetic acid accumulating in the system may be more efficiently removed in this process than in a process of removing acetic acid only in the upper portion of the absorption column, thereby minimizing a loss amount of (meth)acrylic acid. Furthermore, process flexibility may be secured in this process as compared with the case of attempting to discharge the entire amount of acetic acid in the system to the upper portion of the absorption column.

The method for preparation of (meth)acrylic acid according to an exemplary embodiment of the present invention may include supplying the lower discharge stream 420 of the solvent purification column to the crystallizer 500 and obtaining (meth)acrylic acid crystallized in the crystallizer 500.

The (meth)acrylic acid which is crystallized in the crystallizer may be referred to as purified (meth)acrylic acid in some cases, in the present specification. The crystallization process may be performed under common conditions.

The crystallization method for obtaining a product by crystallization in the present invention may be suspension crystallization and layer crystallization without limitation, may be continuous or batchwise, and may be performed in one stage or two stages or more. As a non-limited example, the (meth)acrylic acid may be dynamically crystallized and provided as purified (meth)acrylic acid.

Specifically, in order to dynamically crystallize the (meth)acrylic acid before crystallization, the lower discharge stream of the solvent purification column may be first allowed to flow on a tube inner wall in a falling film form. Further, the temperature of the tube may be adjusted to a freezing point of (meth)acrylic acid or lower to form a crystal in the tube inner wall. Then, the temperature of the tube may be raised to near the freezing point of (meth)acrylic acid to sweat about 5 wt% of (meth)acrylic acid. Further, the mother liquor sweated from the tube is removed and the crystal formed in the tube inner wall is recovered, thereby obtaining the high-purity purified (meth)acrylic acid. The mother liquor may refer to a solution remaining after removing the purified (meth)acrylic acid from the lower discharge stream of the solvent purification column introduced to the crystallizer 500. Therefore, the mother liquor may include a trace amount of (meth)acrylic acid and the extraction solvent, and high-boiling point by-products, in which the (meth)acrylic acid is residual (meth)acrylic acid which is not crystallized in the crystallizer 500, and may be (meth)acrylic acid which is separated in the high-boiling point by-product separation column 600 described later and circulated to the solvent purification column 400 again.

Separation of the mother liquor and the crystallized (meth)acrylic acid may be performed using a solid-liquid separation device, for example, a belt filter, a centrifuge, and the like. The purified (meth)acrylic acid may be recovered as a (meth)acrylic acid recovery stream 510, and the mother liquor may be discharged from the crystallizer 500 through a mother liquor recovery line 520 and supplied to the high-boiling point by-product separation column 600.

That is, the mother liquor may be distilled by the high-boiling point by-product separation column 600 to be separated into a lower fraction including high-boiling point by-products and an upper fraction of the high-boiling point by-products from which high-boiling point by-products have been removed.

The upper fraction of the high-boiling point by-product separation column may include 30 to 60 wt% of the extraction solvent, 40 to 70 wt% of the (meth)acrylic acid, and a residual amount of by-products. That is, as such, the upper fraction includes the extraction solvent which may be recycled and further recovered and (meth)acrylic acid as most components.

According to an exemplary embodiment of the present invention, the upper fraction of the high-boiling point by-product separation column 600 may be discharged through the upper discharge stream 610 of the high-boiling point by-product separation column and supplied to the solvent purification column 400. Thus, the extraction solvent may be recycled and loss of (meth)acrylic acid may be minimized.

Hereinafter, the present invention will be described in more detail by the examples. However, the following examples are provided for illustrating the present invention, and it is apparent to a person skilled in the art that various modifications and alterations may be made without departing from the scope and spirit of the present invention, and the scope of the present invention is not limited thereto.

### Example 1

According to the process flow illustrated in FIG. 1, a preparation process of (meth)acrylic acid was simulated, using an Aspen Plus simulator available from Aspen.

Specifically, a reaction gas including air and a raw material compound (propylene) was supplied to a reactor 10 provided with a catalyst through a reaction gas supply line 1, and a recirculation gas derived from a cooling tower 20 was supplied to the reactor 10 through a recirculation gas transfer line 4. A mixed gas 2 having a composition including (meth)acrylic acid (6.6 mol%), water (16.4 mol%), a high-boiling point material (0.09 mol%), and inert gas (76.3 mol%) was obtained by a gas phase oxidation reaction performed in the reactor 10.

The mixed gas 2 was added to a 11th stage from the top of an absorption column 100 at a temperature of 164°C. In the absorption column 100, the mixed gas was brought into contact with an absorption solvent (water) to obtain a (meth)acrylic acid aqueous solution. The water added to the absorption column 100 was supplied through a lower discharge stream 6 of the cooling tower and a water layer from a layer separator described later, and the water was supplied to the absorption column 100 at a flow rate of 10.6 wt% to the mass flow rate of the mixed gas 2. At this time, the pressure of the upper portion of the absorption column 100 was 1.1 bar, and a temperature of the lower portion of the absorption column 100 was 73.3°C.

Meanwhile, in the absorption column 100, a non-condensable gas including components which were not dissolved in water was separated as the upper discharge stream 110 of the absorption column, and a part 3 of the upper discharge stream 110 of the absorption column was supplied to the cooling tower 20 and the rest was discharged out of the system.

In the cooling tower 20, the non-condensable gas included in the part 3 of the upper discharge stream of the absorption column was dissolved in water. At this time, the water was supplied through a water supply line 5. In the cooling tower 20, the gas which was not dissolved in water was supplied to the reactor 10 through a recirculation gas transfer line 4, and the lower discharge stream 6 of the cooling tower including water and components dissolved in water (acetic acid and (meth)acrylic acid which was not dissolved in water in the absorption column) was supplied to the upper portion of the absorption column 100.

Meanwhile, the (meth)acrylic acid aqueous solution described above was supplied to a degassing column 150 as the lower discharge stream 120 of the absorption column and degassed, and low-boiling point by-products were supplied to the absorption column 100 as an upper discharge stream of the degassing column and a (meth)acrylic acid aqueous solution in which the low-boiling point by-products were degassed was supplied to the extraction column 300 as the lower discharge stream 160 of the degassing column. At this time, the upper discharge stream of the degassing column was supplied to the absorption column 100 at a flow rate of 13.6 wt% with respect to the mass flow rate of water added to the absorption column 100. Meanwhile, the composition of the lower discharge stream 160 of the degassing column included (meth)acrylic acid (64.3 wt%), acetic acid (2.7 wt%), water (32.2 wt%), furfural (0.5 wt%), and maleic acid (0.6 wt%). At this time, the flow rate of the lower discharge stream 160 of the degassing column was 2.3 times the mass flow rate of water supplied to the absorption column 100.

A raffinate stream 320 including water and the extract stream 310 including toluene and (meth)acrylic acid were obtained, by an extraction process performed in the presence of an extraction solvent (toluene) in the extraction column 300. At this time, the extraction solvent (toluene) supplied to the lower portion of the extraction column 300 was supplied at a flow rate of 4 times the mass flow rate of water in the lower discharge stream 160 of the degassing column supplied to the extraction column 300. The raffinate stream 320 was supplied to a layer separator 450 described later, and the extract stream 310 was supplied to the solvent purification column 400.

An upper discharge stream 410 of the solvent purification column including toluene, water, and acetic acid and a lower discharge stream 420 of the solvent purification column including toluene, (meth)acrylic acid, and high-boiling point by-products were obtained by distillation in the solvent purification column 400. At this time, toluene supplied to the solvent purification column 400 was supplied to the upper portion of the solvent purification column 400 at a flow rate of 0.66 times the mass flow rate of toluene supplied to the extraction column 300. Meanwhile, the lower discharge stream 420 of the solvent purification column included (meth)acrylic acid (92.2 wt%), acetic acid (1.2 wt%), toluene (5.1 wt%), furfural (0.7 wt%), and maleic acid (0.5 wt%).

The upper discharge stream 410 of the solvent purification column was supplied to a layer separator 450 and separated into a water layer 470 including water and acetic acid and an organic layer 460 including toluene. The concentration of (meth)acrylic acid in the water layer 470 was 1.56 wt%, and a part of the water layer was supplied to the absorption column 100 and the rest was discharged out of the system as waste water. Meanwhile, a part of the organic layer was supplied to the solvent purification column 400 and the rest was supplied to the extraction column 300. A ratio of the mass flow rate of toluene in the part of the organic layer 460 supplied to the solvent purification column 400 to the mass flow rate of toluene in the upper discharge stream 410 of the solvent purification column was 0.4.

Meanwhile, the lower discharge stream 420 of the solvent purification column described above was supplied and crystallized with the crystallizer 500 to finally obtain (meth)acrylic acid from a (meth)acrylic acid recovery stream 510 including (meth)acrylic acid and the mother liquor was supplied to the high-boiling point by-product separation column 600 through a mother liquor recovery line 520. The content of the (meth)acrylic acid included in the (meth)acrylic acid recovery stream 510 was 99.5 wt% or more.

The mother liquor was distilled in the high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid (54.2 wt%), toluene (37.3 wt%), and residual by-products. The upper discharge stream 610 of the high-boiling point by-product separation column was supplied to the solvent purification column 400.

As a result, energy used in the solvent purification column 400 was 426.4 kcal/kg AA, energy used in the high-boiling point by-product separation column 600 was 36.8 kcal/kg AA, energy used in the crystallizer 500 was 144.5 kcal/kg AA, and thus, a total of 607.7 kcal/kg AA of energy was used.

### Example 2

In Example 2, as compared with Example 1, toluene supplied to the solvent purification column 400 was supplied to the upper portion of the solvent purification column 400 at a flow rate of 0.67 times the mass flow rate of toluene supplied to the extraction column 300, and the lower discharge stream 420 of the solvent purification column included (meth)acrylic acid (87.3 wt%), acetic acid (1.3 wt%), toluene (10.1 wt%), furfural (0.6 wt%), and maleic acid (0.7 wt%) and the content of extraction solvent (toluene) in the lower discharge stream 420 of the solvent purification column was controlled to 10.1 wt%.

As a result, 99.5 wt% or more of (meth)acrylic acid was obtained from the (meth)acrylic acid recovery stream 510 of the crystallizer.

In addition, energy used in the solvent purification column 400 was 431.4 kcal/kg AA, energy used in the high-boiling point by-product separation column 600 was 78.2 kcal/kg AA, energy used in the crystallizer 500 was 144.5 kcal/kg AA, and thus, a total of 654.1 kcal/kg AA of energy was used.

### Comparative Example 1

According to the process flow illustrated in FIG. 2, a preparation process of (meth)acrylic acid was simulated, using the Aspen Plus simulator available from Aspen.

Specifically, in Comparative Example 1, the mixed gas 2 obtained by the same process as in Example 1 was added to the absorption column 100 of the same conditions (operating conditions and the flow rate of water added to the absorption column), and the mixed gas was brought into contact with water in the absorption column 100 to obtain the (meth)acrylic acid aqueous solution. The (meth)acrylic acid aqueous solution was supplied to the degassing column 150 to obtain an upper discharge stream of the degassing column including low-boiling point by-products and a lower discharge stream 160 of the degassing column including the (meth)acrylic acid aqueous solution from which the low-boiling point by-products had been removed. At this time, the upper discharge stream from the degassing column had a flow rate of 13.6 wt% as compared with the mass flow rate of water added to the absorption column, and the lower discharge stream 160 of the degassing column had a flow rate of 2.3 times the mass flow rate of water added to the absorption column. The lower discharge stream 160 of the degassing column included (meth)acrylic acid (64.3 wt%), acetic acid (2.7 wt%), water (31.9 wt%), furfural (0.5 wt%), and maleic acid (0.6 wt%), and the lower discharge stream 160 of the degassing column was supplied to the water separation column 700.

In the water separation column 700, an upper discharge stream 710 of the water separation column including toluene, water, and acetic acid and a lower discharge stream 720 of the water separation column including (meth)acrylic acid and high-boiling point by-products were obtained, by azeotropic distillation performed in the presence of a azeotropic solvent (toluene). The lower discharge stream 720 of the water separation column included (meth)acrylic acid (98.2 wt%), acetic acid (0.08 wt%), toluene (0.0 wt%), furfural (0.81 wt%), and maleic acid (0.93 wt%). At this time, the azeotropic solvent was supplied to the upper portion of the water separation column 700 at a flow rate of 2.1 times the mass flow rate of the lower discharge stream 160 of the degassing column.

The upper discharge stream 710 of the water separation column was supplied to a layer separator 450 and separated into a water layer 470 including water and acetic acid and an organic layer 460 including toluene. A part of the water layer was supplied to the absorption column 100 and the rest was discharged out of the system as waste water. Meanwhile, the organic layer 460 was circulated to the water separation column 700.

Meanwhile, the lower discharge stream 720 of the water separation column was supplied to a high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid. The content of (meth)acrylic acid in the upper discharge stream 610 of the high-boiling point by-product separation column was 99.5 wt% or more, and 99.5 wt% or more (meth)acrylic acid was obtained through the upper discharge stream 610 of the high-boiling point by-product separation column.

As a result, energy used in the water separation column 700 was 683.6 kcal/kg AA and energy used in the high-boiling point by-product separation column 600 was 179.5 kcal/kg AA, and thus, a total of 863.1 kcal/kg AA of energy was used.

### Comparative Example 2

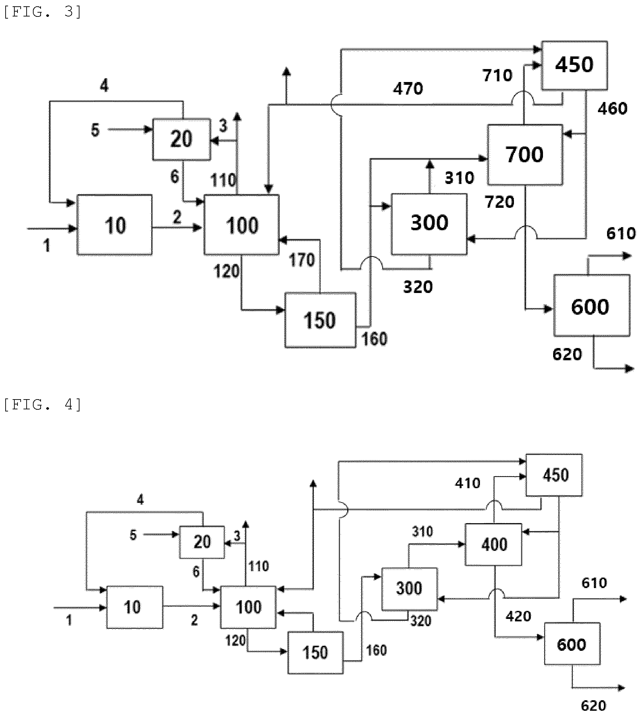

According to the process flow illustrated in FIG. 3, a preparation process of (meth)acrylic acid was simulated, using the Aspen Plus simulator available from Aspen.

In Comparative Example 2, the lower discharge stream 160 of the degassing column having the same composition as Comparative Example 1 was branched at 35 wt% and 65 wt% of the total weight of the lower discharge stream 160 of the degassing column, and supplied to the extraction column 300 and the water separation column 700, respectively.

A raffinate stream 320 including water and the extract stream 310 including toluene and (meth)acrylic acid were obtained, by an extraction process performed in the presence of an extraction solvent (toluene) in the extraction column 300. At this time, the extraction solvent was supplied to the lower portion of the extraction column 300 at a flow rate of 4.1 times the mass flow rate of water in the lower discharge stream of the degassing column which was branched to the upper portion of the extraction column 300 and supplied. The raffinate stream 320 was supplied to the layer separator 450, and the extract stream 310 was mixed with the lower discharge stream of the degassing column which was branched to the water separation column 700 and supplied and supplied to the water separation column 700.

In the water separation column 700, an upper discharge stream 710 of the water separation column including toluene, water, and acetic acid and a lower discharge stream 720 of the water separation column including (meth)acrylic acid and high-boiling point by-products were obtained, by azeotropic distillation performed in the presence of an azeotropic solvent (toluene). The lower discharge stream 720 of the water separation column included (meth)acrylic acid (98.2 wt%), acetic acid (0.1 wt%), toluene (0.0 wt%), furfural (0.81 wt%), and maleic acid (0.93 wt%). At this time, the azeotropic solvent was supplied to the upper portion of the water separation column 700 at a flow rate of 2.1 times the mass flow rate of the extraction solvent supplied to the extraction column.

The upper discharge stream 710 of the water separation column was supplied to a layer separator 450 to obtain a water layer 470 including water and acetic acid and an organic layer 460 including toluene. A part of the water layer was supplied to the absorption column 100 and the rest was discharged out of the system as waste water. Meanwhile, the organic layer 460 was supplied to the water separation column 700 and the extraction column 300.

Meanwhile, the lower discharge stream 720 of the water separation column was supplied to a high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid. The content of (meth)acrylic acid in the upper discharge stream 610 of the high-boiling point by-product separation column was 99.5 wt% or more, and 99.5 wt% or more (meth)acrylic acid was obtained through the upper discharge stream 610 of the high-boiling point by-product separation column.

As a result, energy used in the water separation column 700 was 474.5 kcal/kg AA and energy used in the high-boiling point by-product separation column 600 was 179.5 kcal/kg AA, and thus, a total of 654 kcal/kg AA of energy was used.

### Comparative Example 3

According to the process flow illustrated in FIG. 4, a preparation process of (meth)acrylic acid was simulated, using the Aspen Plus simulator available from Aspen.

In Comparative Example 3, the mixed gas 2 obtained by the same process as in Example 1 was added to the absorption column 100 of the same conditions (operating conditions and the flow rate of water added to the absorption column) as in Example 1, and the mixed gas was brought into contact with water in the absorption column 100 to obtain the (meth)acrylic acid aqueous solution. The (meth)acrylic acid aqueous solution was supplied to the degassing column 150 to obtain an upper discharge stream of the degassing column including low-boiling point by-products and a lower discharge stream 160 of the degassing column including the (meth)acrylic acid aqueous solution from which the low-boiling point by-products had been removed.

The lower discharge stream 160 of the degassing column was supplied to the extraction column 300. A raffinate stream 320 including water and the extract stream 310 including toluene and (meth)acrylic acid were obtained, by an extraction process performed in the presence of an extraction solvent (toluene) in the extraction column 300.

The raffinate stream 320 was supplied to the layer separator 450 described later, and the extract stream 310 was supplied to the solvent purification column 400.

An upper discharge stream 410 of the solvent purification column including toluene, water, and acetic acid and a lower discharge stream 420 of the solvent purification column including (meth)acrylic acid and high-boiling point by-products were obtained by distillation in the solvent purification column 400. At this time, toluene supplied to the solvent purification column 400 was supplied to the upper portion of the solvent purification column 400 at a flow rate of 1.75 times the mass flow rate of toluene supplied to the extraction column 300. Meanwhile, the lower discharge stream 420 of the solvent purification column included (meth)acrylic acid (92.2 wt%), acetic acid (0.1 wt%), toluene (0.01 wt%), furfural (0.8 wt%), and maleic acid (0.9 wt%).

The upper discharge stream 410 of the solvent purification column was supplied to a layer separator 450 and was separated into a water layer including water and acetic acid and an organic layer including toluene. The concentration of (meth)acrylic acid in the water layer was 1.54 wt%, and a part of the water layer was supplied to the absorption column 100 and the rest was discharged out of the system as waste water. Meanwhile, the organic layer was supplied to the extraction column 300 and the solvent purification column 400.

Meanwhile, the lower discharge stream 420 of the solvent purification column was supplied to a high-boiling point by-product separation column 600 to obtain a lower discharge stream 620 of the high-boiling point by-product separation column including high-boiling point by-products and an upper discharge stream 610 of the high-boiling point by-product separation column including (meth)acrylic acid. The content of (meth)acrylic acid in the upper discharge stream 610 of the high-boiling point by-product separation column was 99.5 wt% or more, and finally, 99.5 wt% or more (meth)acrylic acid was obtained therefrom.

As a result, energy used in the solvent purification column 400 was 658.2 kcal/kg AA and energy used in the high-boiling point by-product separation column 600 was 179.5 kcal/kg AA, and thus, a total of 837.7 kcal/kg AA of energy was used.

In Comparative Example 3, distillation in the solvent purification column was performed so that a relatively small amount of the extraction solvent (toluene) was included in the lower discharge stream of the solvent purification column, as compared with Example 1, and though the crystallizer was not provided as in Comparative Examples 1 and 2, 99.5 wt% or more of (meth)acrylic acid was able to be obtained through the high-boiling point by-product separation column, but it was confirmed that total energy used in the process for performing Comparative Example 3 was significantly increased as compared with the examples.

## Claims

1. A method for preparation of (meth)acrylic acid, the method comprising:
bringing a mixed gas including (meth)acrylic acid into contact with water in an absorption column to obtain a (meth)acrylic acid aqueous solution;
supplying the (meth)acrylic acid aqueous solution to an extraction column, bringing the (meth)acrylic acid aqueous solution into contact with an extraction solvent to perform extraction, and obtaining an extract including the (meth)acrylic acid and the extraction solvent;
supplying the extract to a solvent purification column and separating the extract into a lower discharge stream of the solvent purification column including (meth)acrylic acid and an upper discharge stream of the solvent purification column including the extraction solvent in the solvent purification column;
supplying the lower discharge stream of the solvent purification column to a crystallizer and obtaining crystallized (meth)acrylic acid in the crystallizer; and
supplying a mother liquor to a high-boiling point by-product separation column after the crystallization and circulating an upper discharge stream of the high-boiling point by-product separation column from which high-boiling point by-products have been removed to the solvent purification column.

2. The method for preparation of (meth)acrylic acid of claim 1, wherein the (meth)acrylic acid aqueous solution is supplied to a degassing column, and a lower discharge stream of the degassing column from which low-boiling point by-products have been removed is supplied to the extraction column.

3. The method for preparation of (meth)acrylic acid of claim 1, wherein a content of (meth)acrylic acid included in the lower discharge stream of the solvent purification column is 90 to 95 wt%.

4. The method for preparation of (meth)acrylic acid of claim 1,
wherein the lower discharge stream of the solvent purification column includes the extraction solvent, and
a content of the extraction solvent included in the lower discharge stream of the solvent purification column is 1 to 10 wt%.

5. The method for preparation of (meth)acrylic acid of claim 1,
wherein a raffinate including water produced by the extraction and the upper discharge stream of the solvent purification column are supplied to a layer separator and separated into an organic layer including the extraction solvent and a water layer including water and acetic acid, and
a part of the water layer is circulated to the absorption column and the rest is discharged as waste water.

6. The method for preparation of (meth)acrylic acid of claim 1, wherein the upper discharge stream of the high-boiling point by-product separation column includes 30 to 60 wt% of the extraction solvent, 40 to 70 wt% of the (meth)acrylic acid, and a residual amount of by-products.

7. The method for preparation of (meth)acrylic acid of claim 1, wherein a flow rate ratio of acetic acid discharged to an upper portion of the absorption column is 30 wt% to 60 wt%, based on a flow rate of the acetic acid introduced to the absorption column.

8. The method for preparation of (meth)acrylic acid of claim 1,
wherein the mixed gas including (meth)acrylic acid is a reaction product by a gas phase oxidation reaction of a reactant including air, a raw material compound, and a recirculation gas in the reactor, and
the recirculation gas is that, after a part of the upper discharge stream of the absorption column is supplied to a cooling tower and cooled, discharged as an upper discharge stream of the cooling tower and circulated to the reactor.

9. The method for preparation of (meth)acrylic acid of claim 5, wherein a part of the organic layer including the extraction solvent is supplied to the solvent purification column and the rest is supplied to the extraction column.

10. The method for preparation of (meth)acrylic acid of claim 9, wherein a ratio of a flow rate of the extraction solvent in a part of an organic layer supplied to the solvent purification column to a flow rate of the extraction solvent in the upper discharge stream of the solvent purification column is 0.3 to 0.6.

11. The method for preparation of (meth)acrylic acid of claim 1, wherein the extraction solvent includes one or more of benzene, toluene, xylene, n-heptane, cycloheptane, and cycloheptene.
